# FASCICULE DE BREVET EUROPEEN

(11) **EP 1 237 537 B1**
(45) Date de publication et mention de la délivrance du brevet: **23.08.2006**
(21) Numéro de dépôt: 00988913.0
(22) Date de dépôt: 14.12.2000
(51) Int. Cl.: A61K 9/00, A61K 9/06, A61K 47/32, A61K 47/34

(54) **COMPOSITION SOUS FORME DE GEL PREVUE POUR RECEVOIR UN PRINCIPE ACTIF EN SOLUTION OU EN SUSPENSION, NOTAMMENT POUR APPLICATION SUR UNE MUQUEUSE ET PROCEDE DE FABRICATION**
GELZUBEREITUNG ENTHALTEND EINEN GELÖSTEN ODER SUSPENDIERTEN WIRKSTOFF,INSBESONDERE ZUR ANWENDUNG AUF EINER SCHLEIMHAUT UND HERSTELLUNGSVERFAHREN
COMPOSITION IN THE FORM OF A GEL FOR RECEIVING AN ACTIVE INGREDIENT IN A SOLUTION OR SUSPENSION, ESPECIALLY FOR APPLICATION ON A MUCOUS MEMBRANE AND METHOD OF PRODUCTION THEREOF

(30) Priorité: 14.12.1999 FR 9915763
(43) Date de publication de la demande: 11.09.2002
(60) Demande divisionnaire: 06300144.0
(73) Titulaire: Ellipse Pharmaceuticals, 33600 Pessac (FR); Ventitech S.A., 1860 Aigle (CH)
(72) Inventeur: AUZERIE, Jack, F-33560 Sainte-Eulalie (FR)
(74) Mandataire: Fantin, Laurent
(86) Numéro de dépôt international: PCT/FR2000/003533
(87) Numéro de publication internationale: WO 2001/043720

(56) Documents cités:
- EP-A- 0 551 626
- WO-A-91/19481
- WO-A-98/50005
- US-A- 5 902 110

## Description

La présente invention concerne une composition sous forme de gel pour la diffusion d'un principe actif et prévue pour être appliquée sur une muqueuse et plus particulièrement pour des applications de traitements vaginaux.

L'invention couvre aussi le procédé d'élaboration de cette composition pour obtenir un produit adapté.

Le problème du traitement des zones sensibles comme les muqueuses est un problème d'application et de maintien des principes actifs sur la zone avec une durabilité suffisante pour assurer l'action de ces principes, sans que cela engendre une gêne pour la personne traitée. De plus, pour être diffusé commercialement, il faut que le produit soit conditionné en multidoses ou en doses uniques et soit d'une grande facilité d'application sur la zone puisqu'elle est difficile d'accès.

C'est le cas de la voie vaginale qui est l'exemple retenu pour expliciter les caractéristiques et les avantages de la présente invention, sans que ce choix reste limité à cette seule muqueuse. Différents médicaments pourront être combinés au gel pour une application topique. Une première application pourrait être les antifongiques.

On sait que les mycoses ont une forte propension à se développer actuellement pour plusieurs raisons et notamment du fait qu'il est impossible de prévenir ces faits par des traitements préalables face aux facteurs favorisants. Ces mycoses sont pour la plupart opportunistes, iatrogènes et surviennent sur un terrain affaibli suite aux thérapies modernes permettant des actions satisfaisantes sur d'autres zones mais qui induisent ces effets secondaires. Elles surviennent aussi à la suite de séjours en milieu hospitalier.

On connaît des traitements efficaces de ces mycoses mais qui restent avant tout locaux avec des composés qui sont essentiellement les dérivés polyéniques et les dérivés azolés ou imidazolés.

De telles molécules sont conditionnées sous des formes solides : comprimés, ovules ou capsules.

Les comprimés ont une action diffuse qui ne permet pas une action ciblée.

Les ovules comme les capsules vaginales ont une action locale mais une diffusion restreinte. De plus, l'acceptabilité reste faible et les résultats sont peu homogènes et difficilement reproductibles.

On connaît aussi des gels mais ceux-ci, au contact de la muqueuse restent sous forme de gel et ont tendance à perdre de leur viscosité, ce qui conduit à des écoulements inconfortables pour les patientes. De plus, la rémanence reste faible, ce qui limite l'action des principes actifs et/ou nécessite un traitement plus long. De plus, le conditionnement du seul produit commercial connu pour le traitement des mycoses vaginales est du type multidoses avec un applicateur gradué qui n'est pas particulièrement satisfaisant pour ce type de pathologie.

Une deuxième application pourrait être des antibiotiques à visée curative des vaginoses bactériennes par exemple l'éconazole et/ou le métronidazole ou un antibiotique de type macrolide.

Une augmentation trop prononcée de la viscosité n'est pas envisageable pour des problèmes de complexité d'application sur des muqueuses difficiles d'accès.

C'est pourquoi le problème n'est pas limité aux applications des traitements vaginaux mais concerne plus généralement toutes les muqueuses difficiles d'accès comme les muqueuses rectale, nasale, gingivale ou jugale.

La présente invention propose une composition qui permet de conserver les facilités d'application des gels, qui assure une bonne imprégnation de la muqueuse traitée, qui est à fort effet rémanent, qui est confortable pour le patient ou la patiente et qui permet la diffusion d'une large palette de principes actifs. En effet, outre les produits thérapeutiques indiqués ci-avant, tous les produits pour lesquels un effet local est recherché, sont susceptibles d'être incorporés dans un gel selon la présente invention. On peut citer les produits décongestionnants, les anti-inflammatoires, les anti-allergiques, les antalgiques ou les anti-prurigineux, les produits hormonaux substitutifs ou anticonceptionnels naturels tels que les oestroprogestatifs ou les anticonceptionnels synthétiques comme le danazol.

Ainsi le principe actif retenu est incorporé dans un gel thermoréversible, bioadhésif.

La caractéristique de ce gel d'être liquide à température ambiante ou du moins à très faible viscosité, permet une diffusion par différents moyens notamment en aérosol par un conditionnement en bombe avec gaz propulseur ou à l'aide d'une valve à poche. Cette application est réalisée sous le contrôle du personnel médical. Les zones lésionnelles sont ainsi atteintes avec précision et le gel, du fait de sa bioadhésivité, subsiste sur la zone suffisamment longtemps pour que le principe médicamenteux fasse effet.

L'art antérieur décrit des gels thermoréversibles, notamment dans les brevets US-A-4 188 373 et CA-A-1072 413.

Dans ces brevets, on utilise des polymères de polyoxyéthylène-polyoxypropylène. Ces gels sont utilisés comme vecteurs de médicaments à déposer sur des muqueuses.

C'est ainsi que des applications ciblées ont été développées en ophtalmologie avec des gels de propriétés différentes, décrites dans les brevets US-A-4 474 761 et US-A-4 692 454.

En outre, les demandes EP-0 551 626 et WO-9 119 481 décrivent des gels thermoréversibles comprenant un polymère gélifiant et un carbomère.

La présente invention propose une préparation susceptible d'être pulvérisée à température ambiante pour une application mieux ciblée et présentant un caractère fortement bioadhésif sur les muqueuses.

Les différentes figures annexées permettent de montrer les courbes utilisées lors de la description qui va suivre d'une composition adaptée à un domaine particulier, ceci de façon non limitative.

Les différentes figures représentent :
- figure 1, une vue d'une courbe de la viscosité d'une composition comparée avec le produit de base, et
- figures 2A et 2B, des chromatogrammes comparés d'une même composition immédiatement après fabrication et après passage pendant un mois à l'étuve à 45°C.

La composition générale comprend la combinaison d'un gélifiant thermoréversible, d'un gélifiant bioadhésif d'un dérivé cellulosique et d'au moins un principe actif.

Le caractère thermoréversible permet de conserver au gel une très faible viscosité permettant de le considérer comme un liquide à température ambiante et une forme visqueuse à température corporelle. La forme liquide avant application facilite la répartition régulière et reproductible à la surface de la muqueuse tandis que la forme plus visqueuse permet une meilleure adhérence à la muqueuse en limitant les écoulements.

Le caractère bioadhésif permet d'améliorer le contact entre le principe actif et la muqueuse, ce qui augmente l'efficacité thérapeutique et la rémanence de l'action en sorte de limiter le nombre d'applications et la durée du traitement en améliorant l'observance. De même, le caractère bioadhésif renforce l'effet de viscosité engendré par la thermoréversibilité du gel et limite encore les éventuels écoulements au point de les supprimer pour la plupart des cas.

Si l'on prend l'application aux traitements des mycoses vaginales, une composition est indiquée ci-après :
- 15% Poloxamère : solubilisant, épaississant, gélifiant thermoréversible. On peut citer dans la famille des poloxamères, c'est-à-dire des copolymères d'oxydes d'éthylène et de propylène, celui qui est commercialisé sous la dénomination Lutrol F127.
- 0,5% Carbomère : gélifiant bioadhésif. Un exemple de produit du commerce satisfaisant de cette famille des polymères d'acide acrylique de masse moléculaire élevée est le Carbopol 5984 dont la viscosité est comprise entre 25 000 et 45 000 centipoises.
- 1% de principe actif, en l'occurrence du nitrate d'éconazole micronisé, < 50µm, qui est un antimycosique connu.
- des agents de conservation tels que du parahydroxybenzoate de méthyl (POBMS) et de propyl (POBPS), sodé à raison respectivement de 0,1 et 0,05%,
- 0,2% d'hydroxyde de sodium, en sorte de neutraliser la solution, et
- qsp d'eau distillée utilisée comme solvant (quantité suffisante pour 100%) car il se solubilise bien dans l'eau contrairement à d'autres comme le butylhydroxyanisole également essayé en formulation.

La fabrication est réalisée de la façon suivante :
- prémélange 1 ; addition d'une partie de l'eau à l'hydroxyde de sodium
- prémélange 2 ; dispersion du principe actif dans un volume d'eau portée à une température de l'ordre de 30 à 35°C, et
- mélange à la défloculeuse du poloxamère, du carbomère et des prémélanges 1 et 2.

On obtient un gel blanc, thermoréversible et homogène à un pH de l'ordre de 5,5, prêt à conditionner.

On note aussi dans la fabrication que l'ordre d' introduction présente une certaine importance car de préférence, on incorpore le principe actif en dernier, après le poloxamère. Ce dernier présente des propriétés solubilisantes qui diminuent la propension du principe actif à former des agrégats de particules et donc à provoquer une sédimentation non appropriée.

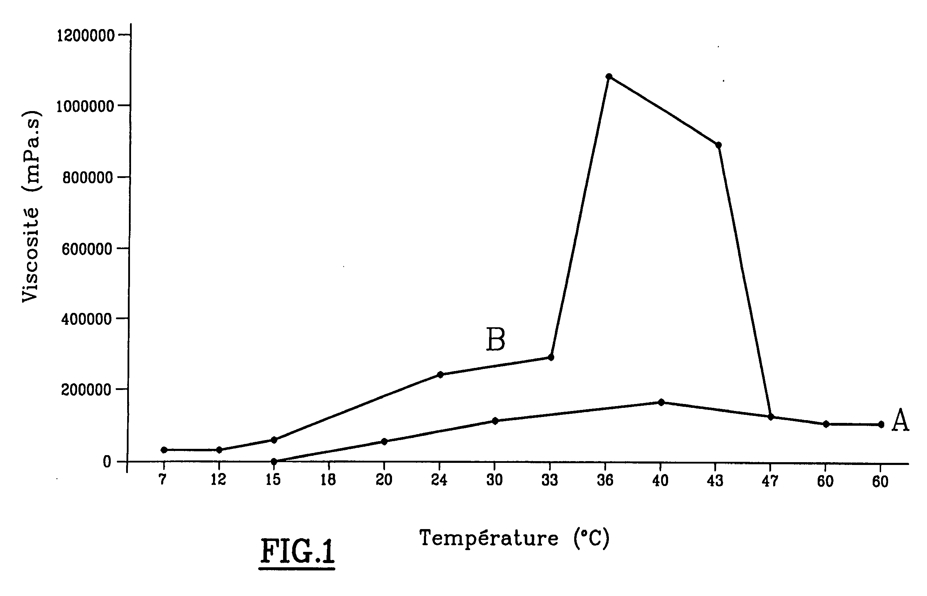

La figure 1 montre la courbe des résultats obtenus comparés concernant les variations de la viscosité en fonction de la température, variations qui sont très significatives. La courbe lissée A est l'image des variations de viscosité du Lutrol F127 à 25% seul et la courbe B est l'image de la brusque augmentation de la viscosité à 33°C de ce même Lutrol en association avec du Carbopol 5984.

On remarque que le Lutrol utilisé de façon isolée n'a pas réellement de comportement thermogélifiant.

Les quantités de poloxamère susceptibles de produire les effets recherchés sont comprises entre 1,0% et 40,0%, plus particulièrement entre 5,0% et 20,0%.

Les quantités de carbomère susceptibles de produire les effets recherchés en association avec le poloxamère pris dans les quantités indiquées ci-dessus sont comprises entre 0,1% et 2,0 plus particulièrement entre 0,5% et 1,0%.

Une autre série d'essais comparatifs consiste à réaliser des tests sur l'oeil de lapin.

On prépare :
- une composition Témoin à base de Lutrol F127 à 25% contenant 0,01% de fluorescéine. La composition présente une viscosité de 20 000 centipoises, à 30°C.
- une composition Test à base de Lutrol F127 à 5% contenant aussi 0,01% de fluorescéine mais avec en plus 0,5% de Carbopol 5984. La composition présente également une viscosité de 20 000 centipoises, à 30°C.

Ces compositions sont appliquées sur l'oeil de cinq lapins, la préparation Témoin sur l'oeil droit et la préparation Test sur l'oeil gauche.

On apprécie la rémanence de la présence de la fluorescéine en examinant la fluorescence des yeux des lapins soumis à un éclairage UV (254nm).

Les résultats indiquent une meilleure rémanence de la présence de la fluorescéine à la surface pour la composition Test :

| Lapin | N°1 | N°2 | N°3 | N°4 | N°5 |
|---|---|---|---|---|---|
| Témoin (Durée en heures) | 6h | 4h | 3h | 2h | 3h |
| Test (Durée en heures) | 9h | 12h | 12h | 10h | 18h |

En effet, l'augmentation de la rémanence est fortement significative puisqu'il existe un rapport 3 à 5 si l'on élimine les extrêmes.

Des essais ont montré l'intérêt d'un ajout d'un dérivé cellulosique tel qu'une cellulose monocristalline commercialisée sous la dénomination "Avicel", agent thixotrope, ou de préférence de l'hydroxypropyl méthylcellulose, commercialisée sous la dénomination "Méthocel E5 premium". Un tel dérivé cellulosique en synergie avec le poloxamère permet de réduire la fourchette de températures de transition et de phase et d'augmenter de façon très conséquente la viscosité du gel lors de la phase de thermogélification, au cours de l'exposition à la chaleur.

Les quantités adaptées varient de 1 à 5% sans que ces limites soient des barrières à l'utilisation mais de fortes quantités ne modifient pas beaucoup les propriétés alors qu'elles peuvent par contre faire disparaître le caractère thermoréversible.

En outre, il convient de ne pas disposer d'un gel trop visqueux à température ambiante car la non fluidité peut nuire à l'application sur la muqueuse d'une part et un tel produit est difficile à débuller après le passage dans les appareils de mélange dynamique d'autre part.

Selon la composition préférée, la conservation est de l'ordre de 5 années de façon stable, c'est à dire que le principe actif reste sous forme dispersée ou solubilisée dans le gel.

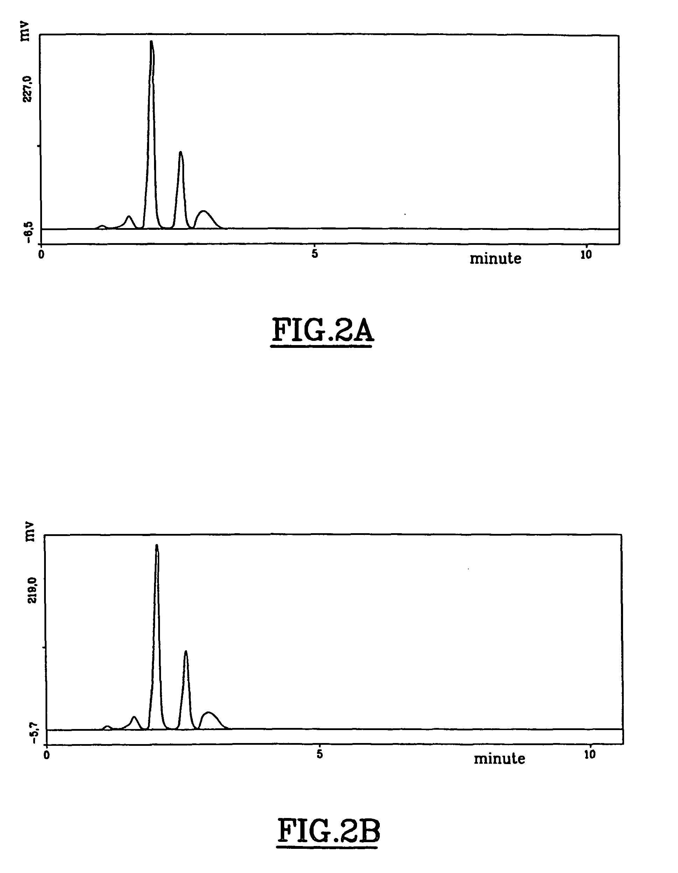

Les courbes des figures 2A et 2B attestent de cette stabilité.

La description qui vient d'être indiquée couvre une application aux traitements des affections de la muqueuse vaginale mais il en serait de même pour toute autre muqueuse d'accès difficile.

Quant aux principes actifs susceptibles d'être associés à cette composition, ils peuvent être de natures différentes et même être associés au sein d'une même composition. On peut ainsi retenir des antifongiques, des antibiotiques ou des produits hormonaux substitutifs ou anticonceptionnels naturels tels les oestroprogestatifs ou des anticonceptionnels synthétiques comme le danazol.

De même, on peut citer les décongestionnants, les anti-inflammatoires, les anti-allergiques, les antalgiques ou les anti-prurigineux.

On peut noter aussi des conséquences importantes et particulièrement avantageuses des compositions qui viennent d'être indiquées.

La faible viscosité à température ambiante permet de recourir à des moyens d'administration particulièrement adaptés et qui utilisent totalement la faible viscosité à température ambiante et la forte bioadhésivité.

Sous contrôle médical, par exemple lors d'un examen gynécologique à l'aide d'un spéculum, à la vue le produit peut être vaporisé ou projeté par le médecin en jets sur la muqueuse atteinte par l'affection à traiter.

Si le médicament à administrer est un antifongique, le gynécologue peut ainsi apporter le produit directement sur la ou les zones infectées.

Le produit adhérant à la muqueuse est à une concentration supérieure aux CMI (concentrations minimales inhibitrices) pendant une durée suffisante pour éradiquer les vecteurs responsables de l'infection.

On peut utiliser la même approche avec un antibiotique traitant des vaginites non spécifiques.

On peut aussi proposer un dispositif multiperforé à introduire dans la cavité naturelle à traiter (vagin, rectum..), ayant une forme anatomique, le produit étant dispensé à travers à ce dispositif par l'intermédiaire d'une valve doseuse ou d'un gaz propulseur.

Il peut aussi y avoir auto-administration.

Les mêmes avantages de la composition selon l'invention se retrouvent quant à la faible viscosité lors de l'application et la forte rémanence une fois appliquée.

## Revendications

1. Composition sous forme de gel prévue pour le traitement d'une muqueuse avec un principe actif, notamment la muqueuse vaginale, comprenant au moins un poloxnmère constituant un gélifiant thermoréversible et au moins un carbomère constituant un produit bioadhésif, **caractérisée en ce qu'**elle comprend également en combinaison :
- au moins un dérivé cellulosique, et
- au moins un principe actif solubilisé ou en suspension.

2. Composition sous forme de gel selon la revendication 1, **caractérisée en ce que** le poloxamère retenu est un copolymère d'oxydes d'éthylène et de propylène.

3. Composition sous forme de gel selon la revendication 1 ou 2, **caractérisée en ce que** le produit bioadhésif est un polymère d'acide acrylique de masse moléculaire élevée.

4. Composition sous forme de gel l'une des revendications 1, 2 ou 3, **caractérisée en ce que** le dérivé cellulosique est l'hydroxypropylméthylcellulose.

5. Composition sous forme de gel selon l'une quelconque des revendications précédentes, **caractérisée en ce que** le principe actif est un antifongique, un antibiotique, des produits hormonaux substitutifs ou anticonceptionnels naturels tels que les oestroprogestatifs ou les anticonceptionnels synthétiques, voire une combinaison de deux ou plusieurs d'entre eux.

6. Composition sous forme de gel selon la revendication 5, **caractérisée en ce que** le principe actif est l'éconazole et/ou le métronidazole.

7. Composition sous forme de gel selon l'une quelconque des revendications précédentes, **caractérisée en ce qu'**elle comprend l'eau comme excipient.

8. Composition sous forme de gel selon l'une quelconque des revendications précédentes, **caractérisée en ce q**u'elle comprend au moins un conservateur et un neutralisant.

9. Composition sous forme de gel selon la revendication 8, **caractérisée en ce que** le conservateur est choisi parmi le parahydrobenzoate de méthyl sodé ou le parahydrobenzoate de propyl sodé.

10. Composition sous forme de gel selon la revendication 8 ou 9, **caractérisée en ce que** le neutralisant est de l'hydroxyde de sodium.

11. Procédé de fabrication de la composition selon l'une quelconque des revendications 7 à 10, **caractérisé en ce qu'**il consiste à réaliser la succession d'étapes suivantes :
- prémélange 1 ; addition d'une partie de l'excipient avec le neutralisant,
- prémélange 2 ; dispersion du principe actif dans l'autre partie d'excipient porté à une température de l'ordre de 30 à 35°C, et
- mélange mécanique du gel thermoréversible, du produit bioadhésif et des prémélanges 1 et 2.

12. Dispositif d'application comprenant la composition sous forme de gel selon la revendication 1, **caractérisé en ce qu'**il comprend des moyens de projection de ladite composition, sous forme d'aérosol ou de jets, en sorte de localiser le produit avec précision sur la zone à traiter.

13. Utilisation d'une composition selon l'une quelconque des revendications 1 à 10, pour l'obtention d'un médicament destiné à être appliqué localement sur une muqueuse en forte concentration sur la zone à traiter.

## Claims

1. Composition in gel form intended for the treatment of a mucosa with an active principle, in particular the vaginal mucosa, comprising at least one poloxamer which constitutes a thermoreversible gelling agent and at least one carbomer which constitutes a bioadhesive product, **characterized in that** it also comprises in combination:
- at least one cellulose derivative, and
- at least one active principle in solution or in suspension.

2. Composition in gel form according to Claim 1, **characterized in that** the poloxamer used is a copolymer of ethylene oxide and propylene oxide.

3. Composition in gel form according to Claim 1 or 2, **characterized in that** the bioadhesive product is an acrylic acid polymer of high molecular weight.

4. Composition in gel form according to one of Claims 1, 2 or 3, **characterized in that** the cellulose derivative is hydroxypropylmethylcellulose.

5. Composition in gel form according to any one of the preceding claims, **characterized in that** the active principle is an antifungal agent, an antibiotic, natural hormone replacement products or contraceptive products such as oestrogen-progestin or synthetic contraceptive products, or a combination of two of more of the above.

6. Composition in gel form according to Claim 5, **characterized in that** the active principle is econazole and/or metronidazole.

7. Composition in gel form according to any one of the preceding claims, **characterized in that** it comprises water as an excipient.

8. Composition in gel form according to any one of the preceding claims, **characterized in that** it comprises at least one preservative and one neutralizer.

9. Composition in gel form according to Claim 8, **characterized in that** the preservative is selected from sodium methyl para-hydroxybenzoate or sodium propyl para-hydroxybenzoate

10. Composition in gel form according to Claim 8 or 9, **characterized in that** the neutralizer is sodium hydroxide.

11. Method for preparing the composition according to any one of Claims 7 to 10, **characterized in that** it comprises carrying out the following steps:
- premix 1: addition of some of the excipient to the neutralizer,
- premix 2: dispersion of the active principle in the rest of the excipient, which has been brought to a temperature of around 30 to 35°C, and
- mechanical mixing of the thermoreversible gel, the bioadhesive product and premixes 1 and 2.

12. Application device comprising the composition in gel form according to Claim 1, **characterized in that** it comprises means for spraying said composition, in aerosol or jet form, so as to localize precisely the product on the area to be treated.

13. Use of a composition according to any one of Claims 1 to 10 to obtain a medicament intended to be applied locally to a mucosa in high concentration on the area to be treated.

## Patentansprüche

1. Gelartige Zusammensetzung zur Behandlung einer Schleimhaut, insbesondere der Vaginalschleimhaut, mit einem Wirkstoff, umfassend mindestens ein thermoreversibles Geliermittel bildendes Poloxamer und mindestens ein bioadhäsives Produkt bildendes Carbomer, **dadurch gekennzeichnet, dass** sie ebenfalls:
- mindestens ein Cellulosederivat und
- mindestens einen gelösten oder suspendierten Wirkstoff in Kombination umfasst.

2. Gelartige Zusammensetzung nach Anspruch 1, **dadurch gekennzeichnet, dass** das ausgewählte Poloxamer ein Copolymer aus Ethylen- und Propylenoxiden ist.

3. Gelartige Zusammensetzung nach Anspruch 1 oder 2, **dadurch gekennzeichnet, dass** das bioadhäsive Produkt ein Acrylsäurepolymer mit einem hohen Molekulargewicht ist.

4. Gelartige Zusammensetzung nach einem der Ansprüche 1, 2 oder 3, **dadurch gekennzeichnet, dass** das Cellulosederivat Hydroxypropylmethylcellulose ist.

5. Gelartige Zusammensetzung nach einem der vorstehenden Ansprüche, **dadurch gekennzeichnet, dass** der Wirkstoff ein Antimykotikum, ein Antibiotikum, natürliche Hormonprodukte zur Substitution oder Empfängnisverhütung wie Östroprogestative oder synthetische Antikonzeptiva, bzw. eine Kombination von zwei oder mehreren von diesen ist.

6. Gelartige Zusammensetzung nach Anspruch 5, **dadurch gekennzeichnet, dass** der Wirkstoff Econazol und/oder Metronidazol ist.

7. Gelartige Zusammensetzung nach einem der vorstehenden Ansprüche, **dadurch gekennzeichnet, dass** sie als Trägerstoff Wasser umfasst.

8. Gelartige Zusammensetzung nach einem der vorstehenden Ansprüche, **dadurch gekennzeichnet, dass** sie mindestens ein Konservierungsmittel und ein Neutralisierungsmittel umfasst.

9. Gelartige Zusammensetzung nach Anspruch 8, **dadurch gekennzeichnet, dass** das Konservierungsmittel zwischen Natrium-Methyl-Parahydroxybenzoat und Natrium-Propyl-Parahydroxybenzoat gewählt ist.

10. Gelartige Zusammensetzung nach Anspruch 8 oder 9, **dadurch gekennzeichnet, dass** das Neutralisierungsmittel Natriumhydroxid ist.

11. Verfahren zur Herstellung der Zusammensetzung nach einem der Ansprüche 7 bis 10, **dadurch gekennzeichnet, dass** es darin besteht, die folgende Schrittfolge durchzuführen:
- Vormischung 1: Zugeben eines Teils des Trägerstoffs mit dem Neutralisierungsmittel,
- Vormischung 2: Dispergieren des Wirkstoffs in den auf eine Temperatur im Bereich von 30 bis 35 °C erwärmten anderen Teil des Trägerstoffs, und
- mechanisches Vermischen des thermoreversiblen Gels, des bioadhäsiven Produktes und der Vormischungen 1 und 2.

12. Applikator umfassend die gelartige Zusammensetzung nach Anspruch 1, **dadurch gekennzeichnet, dass** er Mittel zum Aufspritzen der Zusammensetzung als Strahl oder als Sprühstrahl umfasst, um so das Produkt genau auf den zu behandelnden Bereich aufzubringen.

13. Verwendung einer Zusammensetzung nach einem der Ansprüche 1 bis 10 zur Herstellung eines Arzneimittels zur lokalen Anwendung in hoher Konzentration auf dem zu behandelnden Bereich einer Schleimhaut.
